# EUROPEAN PATENT APPLICATION

(11) **EP 4 847 071 A1**
(43) Date of publication of application: **02.12.2026**
(21) Application number: 26180944.6
(22) Date of filing: 26.05.2026
(51) Int. Cl.: A61M 25/00

(54) **BIOLOGICAL MATERIAL REMOVAL SYSTEM**

(30) Priority: 29.05.2025 US 202563813955 P
(71) Applicant: Abiomed, Inc., Danvers, MA 01923 (US)
(72) Inventor: LEIBOWITZ, Dalia, Danvers, 01923 (US)
(74) Representative: Wittmer, Maximilian

(57) **Abstract**

The present disclosure provides a biological material removal system, comprising: a distal end of a tubular member (204) of a biological material removal system (200), a hub (304) of an access or introducer sheath (300), the tubular member (204) having a lumen (206) extending from a proximal end to a distal end; a coil (202) and a distal end of the coil (202) within or adjacent to biological material (410).

## Description

### CROSS-REFERENCE TO RELATED APPLICATIONS

The present application claims priority to U.S. Provisional Patent Application No. 63/813,955, filed May 29, 2025, titled "Biological Material Removal System and Method", the contents of which are incorporated by reference herein in its entirety.

### FIELD OF INVENTION

The present disclosure relates to medical devices for removing biological material, and more particularly to biological material removal system comprising a coil or fiber (e.g., one or more fibers forming a brush) configured to capture and extract biological material from within an access or introducer sheath.

### BACKGROUND

Intravascular medical procedures have become increasingly common for diagnosing and treating various cardiovascular conditions. These procedures often involve the use of intravascular sheaths to provide access to the patient's vasculature for introducing catheters, guidewires, and other medical devices. Intravascular sheaths typically consist of a hollow tube that is inserted into a blood vessel, allowing for the passage of instruments while maintaining hemostasis.

While intravascular sheaths offer numerous benefits, they can also present challenges related to biological material formation. When blood comes into contact with foreign surfaces, such as the inner walls of an intravascular sheath, it can trigger the coagulation cascade, potentially leading to the formation of biological material. Biological material formation within intravascular sheaths poses risks to patient safety, as the biological material may dislodge and cause complications such as embolism.

### SUMMARY

This summary is provided to introduce a selection of concepts in a simplified form that are further described below in the detailed description. This summary is not intended to identify key features or essential features of the claimed subject matter, nor is it intended to be used as an aid in determining the scope of the claimed subject matter.

According to an aspect of the present disclosure, a method of biological material removal may be provided. The method may include inserting a distal end of a tubular member of a biological material removal system into a hub of an access or introducer sheath, at least partially though a hemostatic valve, the tubular member having a lumen extending from a proximal end to a distal end. The method may include moving a coil distally through the lumen such that a distal end of the coil is within or adjacent to biological material. The method may include capturing the biological material by rotating the coil. The method may include withdrawing the coil and the biological material from the access or introducer sheath.

According to other aspects of the present disclosure, the method may include one or more of the following features. The method may include withdrawing the tubular member from the access or introducer sheath. The method may include providing or selecting a biological material removal system. Moving the coil and rotating the coil may be manually performed. Moving the coil and rotating the coil may be electronically controlled. Moving the coil and rotating the coil may be initiated by pressing a button. When the coil moves distally, the coil may move axially without rotation. When the coil moves distally, the coil may move axially and rotate at the same time. In some instances, the coil may be inserted axially, and then may only move via rotation.

According to another aspect of the present disclosure, a biological material removal system may be provided. The biological material removal system may include a tubular member having a proximal end and a distal end, and a lumen extending therethrough, the tubular member may be configured to extend into an access or introducer sheath. The biological material removal system may include a coil extending at least partially through the lumen, a distal end of the coil may be configured to extend distally beyond a distal end of the tubular member when the coil is fully inserted into the tubular member.

According to other aspects of the present disclosure, the biological material removal system may include one or more of the following features. The biological material removal system may include a handle or grip operably coupled to a proximal end of the coil. The biological material removal system may include a handle or grip operably coupled to a proximal end of the tubular member. The tubular member and coil may be free of mechanical or electrical connections between them. The tubular member and coil may be operably coupled together. The biological material removal system may include a motor or actuator operably coupled to a proximal end of the coil, and may be configured to control axial movement and/or rotation of the coil. The biological material removal system may include a sensor disposed in or on the tubular member. Axial movement and/or rotation of the coil may be controlled based on information from the sensor. The tubular member may be composed of one or more polymeric materials. The coil may be composed of one or more polymer materials. A pitch of the coil may be constant. A first portion of the coil may have a first pitch, and a second portion of the coil may have a second pitch. A distal end of the coil may have a rounded tip. A distal end of the coil may have a tapered tip.

According to another aspect of the present disclosure, a kit may be provided. The kit may include a tubular member having a proximal end and a distal end, and a lumen extending therethrough, the tubular member may be configured to extend into an access or introducer sheath. The kit may include a coil configured to slidably extend through the lumen, a distal end of the coil may be configured to extend distally beyond a distal end of the tubular member when the coil is fully inserted into the tubular member.

According to another aspect of the present disclosure, a method of biological material removal may be provided. **The** method may include inserting a distal end of an elongate member into a hub of an access or introducer sheath through a hemostatic valve. **The** distal end of the elongate member may include a coil or fiber configured to interact with biological material. This may include moving the coil or fiber distally through the hub such the coil or fiber is within or adjacent to biological material. **The** method may include capturing the biological material by rotating the elongate member. **The** method may include withdrawing the elongate member and the biological material from the access or introducer sheath.

According to other aspects of the present disclosure, the method may include one or more of the following features. The method may include inserting a distal end of a tubular member into the hub prior to inserting the elongate member, wherein inserting a distal end of an elongate member into the hub may include inserting the distal end of the elongate member through the tubular member. The method may include withdrawing the tubular member from the hub prior to withdrawing the elongate member. The method may include withdrawing the tubular member from the hub simultaneous with or after withdrawing the elongate member. The method may include providing or selecting a biological material removal system. Rotating the elongate member may be manually performed. Rotating the elongate member may be electronically controlled. Rotating the elongate member may be initiated by pressing a button.

According to another aspect of the present disclosure, a biological material removal device may be provided. The biological material removal device may include an elongated member having at least a distal portion comprised of a shape memory material. The biological material removal device may include a coil or fiber disposed around the exterior of at least some of the distal portion, the coil or fiber may be configured to interact with biological material. The biological material removal device may be configured to be slidably inserted through a hemostatic valve of an access or introducer hub.

According to other aspects of the present disclosure, the biological material removal device may include one or more of the following features. The shape memory material may be nitinol. The coil or fiber may be a polymeric material. The coil or fiber may be a metal. The biological material removal device may include a handle or grip operably coupled to a proximal end of the elongated member. A distal end of the elongated member may be rounded. A distal end of the elongated member may be tapered. A distal end of the elongated member may be configured to be offset axially from the proximal end of the shape memory material. A distal end of the elongated member may be configured to be coplanar with a proximal end of the shape memory material. The tubular member and coil may be free of mechanical or electrical connections between them. The tubular member and coil may be operably coupled together. The biological material removal device may include a motor or actuator operably coupled to a proximal end of the elongate member, and may be configured to control rotation of the elongate member.

According to another aspect of the present disclosure, a kit may be provided. The kit may include a biological material removal device as described above. The kit may include a first tubular member having a proximal end and a distal end, and a lumen extending therethrough, the tubular member may be configured to extend into an access or introducer sheath, the biological material removal device may be configured to be slidably received through the lumen.

According to other aspects of the present disclosure, the kit may include one or more of the following features. The kit may include a coil configured to slidably extend through the lumen, a distal end of the coil may be configured to extend distally beyond a distal end of the tubular member when the coil is fully inserted into the tubular member. The kit may include a biological material removal system including a second tubular member having a proximal end and a distal end, and a lumen extending therethrough, the second tubular member may be configured to extend into an access or introducer sheath. The kit may include a coil configured to extend at least partially through the lumen, a distal end of the coil may be configured to extend distally beyond a distal end of the second tubular member when the coil is fully inserted into the tubular member.

The foregoing general description of the illustrative embodiments and the following detailed description thereof are merely exemplary aspects of the teachings of this disclosure and are not restrictive.

### BRIEF DESCRIPTION OF FIGURES

Non-limiting and non-exhaustive examples are described with reference to the following figures.
FIG. 1 illustrates a flowchart of a method for biological material removal, according to aspects of the present disclosure.
FIG. 2 depicts a section view of a biological material removal system, according to an embodiment.
FIG. 3 shows an exploded view of a portion of a biological material removal system, in accordance with example embodiments.
FIG. 4 illustrates a section view of a biological material removal system showing internal components, according to an aspect of the present disclosure.
FIG. 5 depicts a flowchart of another method for biological material removal, according to an embodiment.
FIG. 6 shows a section view of a biological material removal device, in accordance with example embodiments.
FIG. 7A and FIG. 7B illustrate section views of a biological material removal system, according to aspects of the present disclosure.
FIG. 8 depicts a section view of a biological material removal system with an access sheath, according to an embodiment.
FIG. 9 shows a section view of a biological material removal system interacting with biological material, in accordance with example embodiments.
FIG. 10 illustrates a section view of a biological material removal device assembly, according to aspects of the present disclosure.
FIG. 11A-11C illustrates a technique for using a biological material removal system according to an aspect of the present disclosure.

### DETAILED DESCRIPTION

The following description sets forth exemplary aspects of the present disclosure. It should be recognized, however, that such description is not intended as a limitation on the scope of the present disclosure. Rather, the description also encompasses combinations and modifications to those exemplary aspects described herein.

The present disclosure relates to systems and methods for removing biological material from medical devices such as intravascular access sheaths. The invention provides biological material removal devices that may include coils or fibers configured to capture and extract biological material from within sheaths or hubs.

These systems and methods may address several problems associated with intravascular procedures. For example, biological material formation within access sheaths can pose risks to patient safety if biological material dislodges and cause complications like embolism. The disclosed biological material removal devices may allow for efficient extraction of such biological material before they can cause harm. Additionally, biological material buildup in sheaths may interfere with the insertion and removal of other medical instruments. The ability to clear biological material may help maintain proper function of the access sheath throughout a procedure.

The invention may also provide ways to remove biological material without needing to fully remove and replace the access sheath, which could disrupt ongoing treatments. The biological material removal systems may be designed for compatibility with standard access sheaths and hubs, potentially allowing for seamless integration into existing procedural workflows. Overall, these devices and methods may enhance the safety and efficacy of intravascular procedures by addressing the common issue of biological material formation in medical access devices.

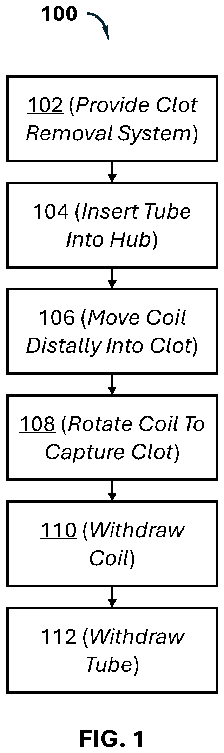

In various aspects, a method for biological material removal from, e.g., an access or introducer sheath, may be provided. Referring to FIG. 1, the method 100 may include providing 102 or selecting a biological material removal system. In some embodiments, the system may be selected based on one or more dimensions or characteristics of the access or introducer sheath the system is being used with.

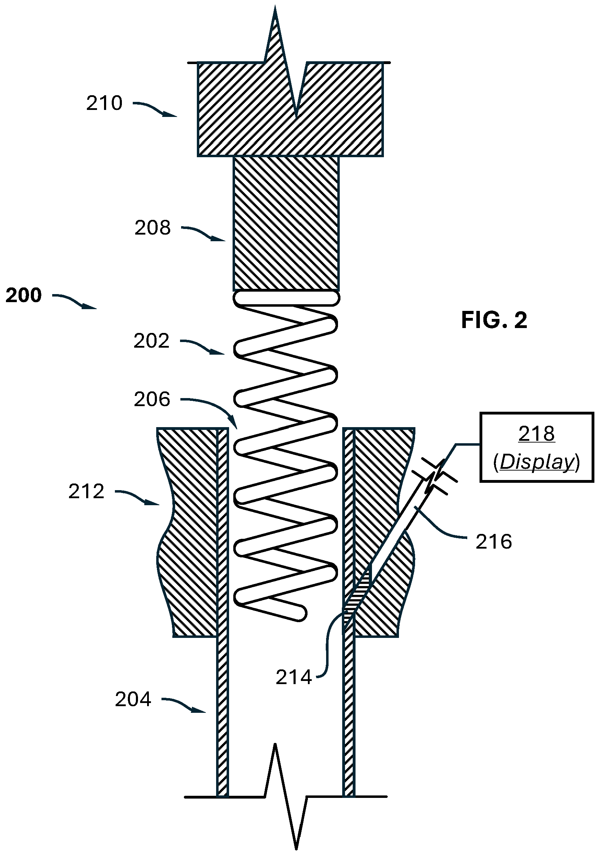

A biological material removal system 200 may include a tubular member 204 and a coil 202. FIG. 2 illustrates a section view of the biological material removal system 200. The tubular member 204 may have a proximal end and a distal end, with a lumen 206 extending therethrough. In some cases, the tubular member 204 may be configured to extend into an access or introducer sheath.

The coil 202 may extend at least partially through the lumen 206 of the tubular member 204. In some cases, a distal end of the coil 202 may be configured to extend distally beyond a distal end of the tubular member 204 when the coil 202 is fully inserted into the tubular member 204. This configuration may allow the coil 202 to interact with biological material within the access or introducer sheath while the tubular member 204 remains in place.

The biological material removal system 200 may be designed to facilitate the removal of biological material from medical devices such as intravascular access sheaths. The tubular member 204 may provide a pathway for the coil 202 to be inserted and manipulated within the access or introducer sheath. The coil 202 may be configured to capture and extract biological material from within the sheath or associated hub.

In some cases, the coil 202 may be slidably disposed within the lumen 206 of the tubular member 204. This arrangement may allow for controlled movement of the coil 202 relative to the tubular member 204 during biological material removal procedures. The ability to extend the coil 202 beyond the distal end of the tubular member 204 may enable targeted interaction with biological material in specific areas of the access or introducer sheath.

The biological material removal system 200 may be provided as part of a kit. In some cases, the kit may include the tubular member 204 and the coil 202 as separate components that can be assembled for use. The kit may also include additional components such as a second tubular member and coil, providing options for different biological material removal scenarios or backup equipment.

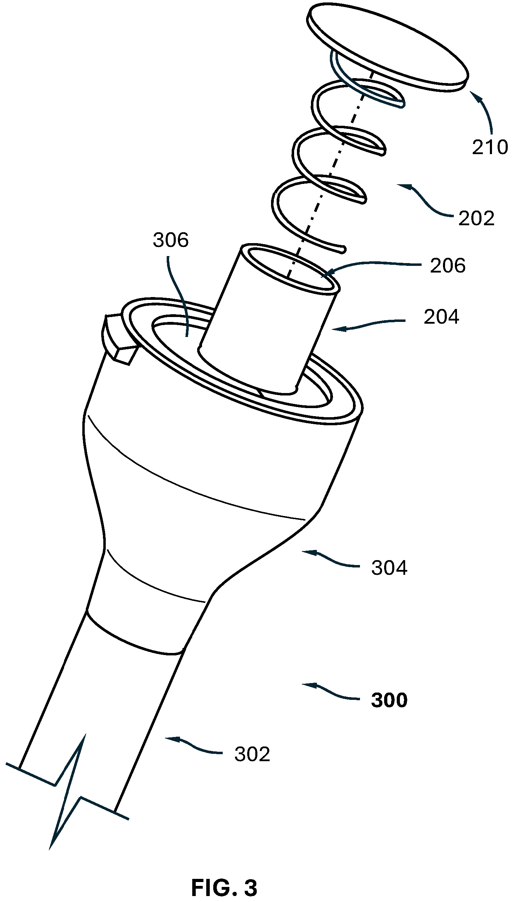

The tubular member 204 may be a key component of the biological material removal system 200. As shown in FIG. 2 and FIG. 3, the tubular member 204 may have an elongated cylindrical structure with a proximal end and a distal end. The lumen 206 may extend through the entire length of the tubular member 204, providing a passageway for other components of the biological material removal system 200.

In some cases, the tubular member 204 may be composed of one or more polymeric materials. This composition may provide flexibility and biocompatibility suitable for intravascular use. The polymeric materials may include, but are not limited to, polyethylene, polyurethane, polytetrafluoroethylene (PTFE), or other medical-grade polymers.

The dimensions of the tubular member 204 may vary depending on the specific application. In some cases, the tubular member 204 may have an outer diameter compatible with standard access or introducer sheaths used in intravascular procedures. The inner diameter of the tubular member 204, which defines the lumen 206, may be sized to accommodate the coil 202 or other elongate members used for biological material removal.

As illustrated in FIG. 3, the tubular member 204 may be configured to be inserted into a hub 304 of an access or introducer sheath 300. In some cases, the distal end of the tubular member 204 may be inserted at least partially through a hemostatic valve 306 within the hub 304. This configuration may allow the tubular member 204 to provide a pathway for other components of the biological material removal system 200 while maintaining hemostasis.

The tubular member 204 may be designed to allow for the passage of dislodged biological material. In some cases, the lumen 206 of the tubular member 204 may provide a channel through which dislodged biological material can exit the hub 304 with the flow of pressurized blood. This feature may facilitate the removal of biological material from the access or introducer sheath 300 without the need for complete withdrawal of the sheath.

In some cases, the tubular member 204 may be inserted into the hub 304 prior to the insertion of an elongate member, such as the coil 202. The elongate member may then be inserted through the lumen 206 of the tubular member 204. This sequence may allow for precise positioning of the biological material removal components within the access or introducer sheath 300.

The tubular member 204 may be designed for easy insertion and withdrawal from the access or introducer sheath 300. In some cases, the tubular member 204 may be withdrawn from the hub 304 prior to the withdrawal of the elongate member. Alternatively, the tubular member 204 may be withdrawn simultaneously with or after the withdrawal of the elongate member. These options may provide flexibility in the biological material removal procedure, allowing for adaptation to different clinical scenarios.

The surface of the tubular member 204 may be treated or coated to enhance its performance. In some cases, the inner surface of the lumen 206 may have a low-friction coating to facilitate smooth movement of the coil 202 or other elongate members. The outer surface of the tubular member 204 may have a coating to improve biocompatibility or reduce the risk of biological material formation.

The coil 202 may be a key component of the biological material removal system 200. As shown in FIG. 2 and FIG. 3, the coil 202 may extend at least partially through the lumen 206 of the tubular member 204. In some cases, the coil 202 may be configured to extend distally beyond the distal end of the tubular member 204 when fully inserted. A central axis of the coil is preferably straight.

The coil 202 may be composed of various materials. In some cases, the coil 202 may be made of one or more polymer materials. Alternatively, the coil 202 may be composed of metal. In some cases, the coil 202 may be made of a shape memory material such as nitinol. The use of shape memory materials may allow the coil 202 to maintain a desired shape or configuration during use.

The structure of the coil 202 may vary. In some cases, the pitch of the coil 202 may be constant along its length. Alternatively, a first portion of the coil 202 may have a first pitch and a second portion of the coil 202 may have a second pitch. This variation in pitch may allow for different functional characteristics along the length of the coil 202.

The distal end of the coil 202 may have different configurations. In some cases, the distal end of the coil 202 may have a rounded tip. Alternatively, the distal end of the coil 202 may have a tapered tip. In some cases, the coil 202 may have a sharp tip that can hook onto biological material.

The coil 202 may be designed to interact with biological material in various ways. In some cases, the coil 202 may be moved distally through the lumen 206 such that the distal end of the coil 202 is within or adjacent to biological material. The coil 202 may then be used to capture the biological material by rotating the coil 202.

The movement of the coil 202 may vary during use. In some cases, when the coil 202 moves distally, the coil 202 may move axially without rotation. Alternatively, when the coil 202 moves distally, the coil 202 may move axially and rotate at the same time. The coil 202 may be swept by twirling the tubular member 204 e.g., three hundred and sixty degrees.

In some cases, the coil 202 may be in the form of a brush. Alternatively, a coil or fiber may be disposed around the exterior of at least some of the distal portion of an elongated member. In various aspects, the elongated member may be, e.g., a solid elongated member or a tubular elongated member. This configuration may enhance the ability of the device to interact with and capture biological material.

The biological material removal system 200 may be designed to allow for the withdrawal of the coil 202 and captured biological material. In some cases, the coil 202 and the biological material may be withdrawn from the access or introducer sheath 300. This withdrawal may occur through the hemostatic valve 306 of the hub 304.

The dimensions and specific configuration of the coil 202 may vary depending on the intended use and the size of the tubular member 204 and access or introducer sheath 300. The coil 202 may be sized to allow for effective biological material capture while still permitting smooth movement through the lumen 206 of the tubular member 204.

The biological material removal system 200 may include handles or grips to facilitate manipulation and control of the coil 202 and the tubular member 204. As shown in FIG. 2, a handle or grip 210 may be operably coupled to a proximal end of the coil 202. For example, in some cases, the handle or grip may be attached directly to a proximal end of the coil. In some cases, the handle or grip may be coupled to a connector portion 208, and a distal portion of the connector portion 208 may be directly coupled to a proximal end of the coil. The connector portion 208 may be configured to be slidably received by the tubular member 204. In some cases, the connector portion 208 may have a same outer diameter as the coil. In some cases, the connector portion 208 may have a larger outer diameter than the coil. In some cases, the connector portion 208 may have a smaller outer diameter than the coil. Additionally, a handle or grip 212 may be operably coupled to a proximal end of the tubular member 204.

The handle or grip 210 associated with the coil 202 may be designed to allow for precise control of the coil's movement and rotation. In some cases, the handle or grip 210 may have a cylindrical shape with textured surfaces to enhance grip. The handle or grip 210 may be composed of materials such as medical-grade plastics or metals that provide durability and ease of sterilization.

Similarly, the handle or grip 212 connected to the tubular member 204 may be configured to facilitate insertion and withdrawal of the tubular member 204 from the access or introducer sheath 300. The handle or grip 212 may have a larger diameter than the tubular member 204 to provide a comfortable grip for the user.

In some cases, the handles or grips 210, 212 may be designed to work in conjunction with each other. For example, the relative positions of the handles or grips 210, 212 may indicate the extent to which the coil 202 extends beyond the distal end of the tubular member 204.

The handles or grips 210, 212 may also incorporate features to enhance functionality. In some cases, the handle or grip 210 may include markings or indicators to show the rotational position of the coil 202. The handle or grip 212 may have depth markers to indicate the insertion depth of the tubular member 204 into the hub 304 of the access or introducer sheath 300. A tubular member 302 may be coupled to a distal end of the hub 304.

The connection between the handles or grips 210, 212 and their respective components may vary. In some cases, the handles or grips 210, 212 may be permanently affixed to the coil 202 and tubular member 204. Alternatively, the handles or grips 210, 212 may be detachable, allowing for replacement or customization.

The shape and size of the handles or grips 210, 212 may be designed to accommodate various hand sizes and gripping preferences. In some cases, the handles or grips 210, 212 may have ergonomic contours to reduce user fatigue during prolonged procedures.

The materials used for the handles or grips 210, 212 may be selected to provide specific properties. In some cases, the handles or grips 210, 212 may be made of materials that offer thermal insulation, reducing heat transfer from the user's hand to the coil 202 or tubular member 204.

The handles or grips 210, 212 may also serve as interfaces for additional components. In some cases, the handle or grip 210 may incorporate a mechanism for locking the coil 202 in place relative to the tubular member 204. The handle or grip 212 may include ports for flushing or aspiration through the lumen 206 of the tubular member 204.

In some cases, the design of the handles or grips 210, 212 may take into account the need to maintain a sterile field during procedures. The handles or grips 210, 212 may have smooth surfaces and minimal crevices to facilitate cleaning and reduce the risk of contamination. In some instances, the device may be disposable. In some instances, the handle or grip may be reuseable while the rest of the device is disposable. Thus, in some instances, the handle or group may be removably coupled to the tubular member.

The positioning of the handles or grips 210, 212 relative to other components of the biological material removal system 200 may be significant. In some cases, the handle or grip 210 may be positioned such that a distal end of the coil 202 is configured to be offset axially from the proximal end of any shape memory material used in the system. Alternatively, the handle or grip 210 may be designed to allow a distal end of the coil 202 to be coplanar with a proximal end of any shape memory material when fully extended.

The handles or grips 210, 212 may also play a role in the overall balance and weight distribution of the biological material removal system 200. In some cases, the handles or grips 210, 212 may be weighted or shaped to provide a counterbalance to the distal portions of the coil 202 and tubular member 204, enhancing overall system stability during use.

In some instances, the system may include one or more sensors 214, and preferably at least one sensor is a camera adapted to providing video showing the interior of, e.g., a sheath hub through, e.g., the lumen of the tubular member, to ensure the biological material has been removed. In some instances, the sensors may be operably coupled to a display 218 (e.g., via one or more wires 216). The wires may extend through the handle or grip 212, or may extend, e.g., only through the tubular member.

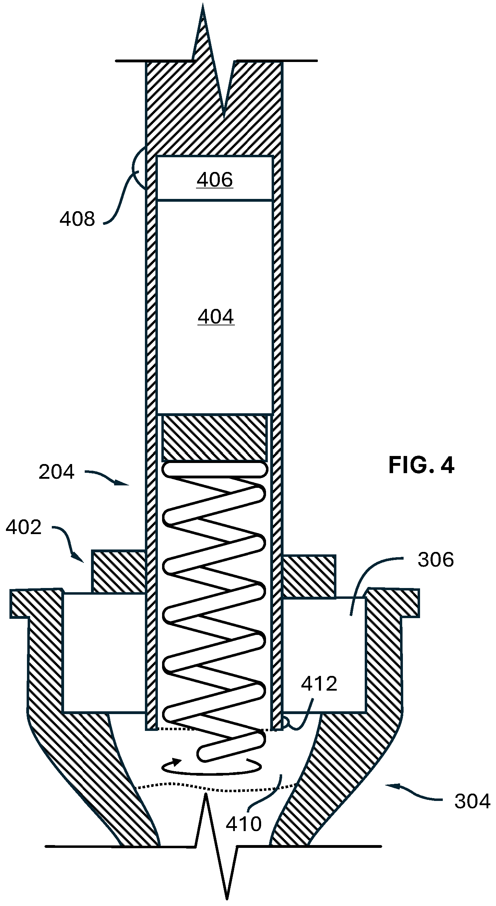

The biological material removal system 200 may include various components for controlling the movement and operation of the coil 202. FIG. 4 illustrates a cross-sectional view of the biological material removal system 200, showing internal components and their arrangement.

In some cases, a motor or actuator 404 may be operably coupled to a proximal end of the coil 202. The motor or actuator 404 may be configured to control axial movement and/or rotation of the coil 202. This configuration may allow for precise and controlled manipulation of the coil 202 during biological material removal procedures.

The biological material removal system 200 may include circuitry 406 associated with the motor or actuator 404. The circuitry 406 may be designed to control the operation of the motor or actuator 404, allowing for electronic control of the coil's movement and rotation. In some cases, the circuitry 406 may be located in proximity to the motor or actuator 404 within the biological material removal system 200.

A button 408 may be incorporated into the biological material removal system 200. The button 408 may be configured to initiate the movement and rotation of the coil 202. In some cases, pressing the button 408 may activate the motor or actuator 404, causing the coil 202 to move axially, rotate, or perform a combination of these movements.

The biological material removal system 200 may include a sensor 412 disposed in or on the tubular member 204. The sensor 412 may be designed to gather information about the environment within the access or introducer sheath 300 or the surrounding tissue. In some cases, the axial movement and/or rotation of the coil 202 may be controlled based on information from the sensor 412. This feedback mechanism may allow for adaptive control of the biological material removal process.

In some cases, the tubular member 204 and coil 202 may be operably coupled together. This coupling may allow for coordinated movement and control of both components. Alternatively, the tubular member 204 and coil 202 may be free of mechanical or electrical connections between them, allowing for independent manipulation.

The motor or actuator 404 may also be operably coupled to a proximal end of an elongate member, such as the coil 202. In this configuration, the motor or actuator 404 may be designed to control rotation of the elongate member. This arrangement may facilitate the capture and removal of biological material by allowing controlled rotational movement of the coil 202 or other biological material-interacting components.

While electronic control may be implemented in some cases, the biological material removal system 200 may also allow for manual operation. In some cases, moving the coil 202 and rotating the coil 202 may be manually performed. This manual option may provide flexibility in different clinical scenarios or serve as a backup in case of electronic system failure.

The integration of these motor and control components may allow for precise manipulation of the coil 202 or other elongate members within the access or introducer sheath 300. The ability to control axial movement and rotation electronically or manually may enhance the effectiveness of biological material removal procedures while potentially reducing procedural complexity and operator fatigue.

The coil may have various characteristics that contribute to its functionality in the biological material removal system. In some cases, the pitch of the coil may be constant along its entire length. This uniform pitch may provide consistent interaction with biological material throughout the coil's length.

Alternatively, the coil may have varying pitch characteristics. In some cases, a first portion of the coil may have a first pitch, and a second portion of the coil may have a second pitch. This variation in pitch may allow for different functional properties along the length of the coil. For example, a tighter pitch in one section may enhance biological material capture, while a looser pitch in another section may facilitate biological material removal.

The distal end of the coil may have different configurations to enhance its effectiveness in interacting with biological material. In some cases, the distal end of the coil may have a rounded tip. This rounded tip may help prevent damage to vessel walls or other delicate tissues during insertion and manipulation of the coil.

Alternatively, the distal end of the coil may have a tapered tip. A tapered tip may facilitate easier insertion of the coil through narrow passages or through biological material. The gradual narrowing of a tapered tip may also help in initiating biological material engagement.

The material composition of the coil may vary depending on the specific requirements of the biological material removal system. In some cases, the coil may be composed of one or more polymer materials. Polymer materials may offer flexibility and biocompatibility suitable for intravascular use.

In other cases, the coil may be composed of metal. Metallic coils may provide different mechanical properties, such as increased rigidity or radiopacity, which may be beneficial in certain clinical scenarios.

The specific dimensions, pitch variations, tip designs, and material compositions of the coil may be selected based on factors such as the target vessel size, biological material characteristics, and desired biological material removal mechanism. These characteristics may be optimized to enhance the overall performance and safety of the biological material removal system.

Referring again to FIG. 1, the method may include inserting 104 a distal end of the tubular member 204 into a hub 304 of an access or introducer sheath 300, at least partially though a hemostatic valve 306.

The method may include moving 106 a coil distally through the lumen (i.e., so at least some of the coil extends beyond the distal end of the lumen), such that a distal end of the coil is within or adjacent to biological material 410. This may be done in various ways, including by pushing the coil (e.g., using a handle or grip 210 operably coupled to the coil) distally through the lumen, by having a motor or actuator translate the coil axially, etc. The coil may move axially without rotation, or, in some cases, the coil may rotate as it moves axially.

The method may include capturing 108 the biological material by rotating the coil. This may be done in various ways, including by rotating the coil around a central axis by hand, by having a motor rotate the coil, etc.

The method may include withdrawing 110 the coil and the biological material from the access or introducer sheath. That is, once biological material has been captured by the coil (and the device can capture one or more separate pieces of biological material), the biological material can be removed by removing the coil from the hub of the access tube. This can be done moving the coil proximally by hand, by having a motor or actuator translate the coil accordingly, etc.

The method may include withdrawing 112 the tube from the access or introducer sheath. In some aspects, this is done at the while also removing the coil. In some aspects, this may be done after the coil has been removed.

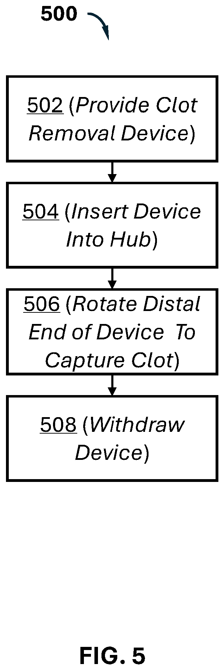

Another method may be used, in addition to the disclosed method 100 (e.g., after performing method 100), or as an alternative to performing that method. Specifically, referring to FIG. 5, the method for biological material removal 500 may include providing 502 or selecting a biological material removal device.

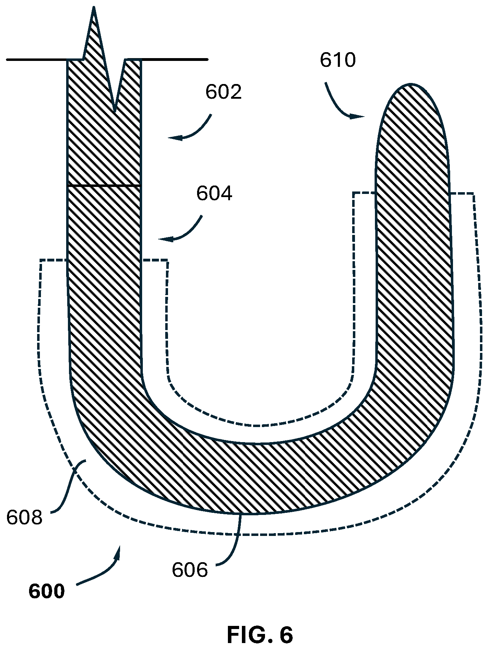

FIG. 6 illustrates a section view of a biological material removal device 600. The biological material removal device 600 may be part of a biological material removal system designed for removing biological material from medical devices such as intravascular access sheaths.

The biological material removal device 600 may include an elongated member having a proximal portion 602 and at least a distal portion comprised of a shape memory material 604. In some cases, the shape memory material 604 may allow the distal portion of the elongated member to maintain a desired shape or configuration during use.

An exterior surface 606 of the elongated member may include a coil or fiber 608 disposed around at least some of the distal portion. The coil or fiber 608 may be configured to interact with biological material, facilitating their capture and removal.

In some instances, the fiber may be one or more free-standing fiber, coupled to device at one end of the fiber. In some instances, the fibers may form a brush or have a brush-like configuration. In some instances, the fibers may extend radially outward from the elongated member. In some instances, the fibers may extend circumferentially around at least some of the elongated member. In some instances, only a single fiber is present. In some instances, multiple fibers are present.

The biological material removal device 600 may have a distal end 610, which may be designed to interface with biological material within an access or introducer sheath. In some cases, the distal end 610 may extend beyond the distal end of the tubular member when the biological material removal device 600 is fully inserted into the tubular member.

The biological material removal device 600 may be configured to be slidably inserted through a hemostatic valve of an access or introducer hub. This configuration may allow the biological material removal device 600 to be used in conjunction with existing medical access devices while maintaining hemostasis.

In some cases, the biological material removal system may include the biological material removal device 600 in combination with other components. For example, the biological material removal system may comprise a tubular member with a proximal end, distal end, and lumen extending therethrough. The tubular member may be configured to extend into an access or introducer sheath, providing a pathway for the biological material removal device 600.

The coil or fiber 608 of the biological material removal device 600 may extend at least partially through the lumen of the tubular member. In some cases, the distal end of the coil or fiber 608 may be configured to extend distally beyond the distal end of the tubular member when fully inserted. This arrangement may allow the coil or fiber 608 to interact with biological material within the access or introducer sheath while the tubular member remains in place.

In some cases, providing or selecting a biological material removal system may involve choosing appropriate components based on the specific clinical scenario. The selection may take into account factors such as the size of the access or introducer sheath, the location and characteristics of the biological material, and the desired method of biological material removal.

The biological material removal device 600 may include an elongated member with at least a distal portion comprised of a shape memory material 604. In some cases, the shape memory material 604 may be nitinol. The use of shape memory materials like nitinol may allow the distal portion of the elongated member to maintain a desired shape or configuration during use, enhancing the device's ability to navigate through and interact with biological material.

A coil or fiber 608 may be disposed around the exterior surface 606 of at least some of the distal portion of the elongated member. The coil or fiber 608 may be configured to interact with biological material, facilitating their capture and removal. In some cases, the coil or fiber 608 may be composed of one or more polymer materials. Alternatively, the coil or fiber 608 may be a metal. The choice of material for the coil or fiber 608 may depend on factors such as desired flexibility, durability, and interaction with biological material.

The configuration of the coil or fiber 608 may vary. In some cases, the coil or fiber 608 may be in the form of a brush made of shape memory material. This brush-like configuration may enhance the device's ability to capture and retain biological material during removal procedures.

The pitch of the coil or fiber 608 may be designed to optimize biological material capture and removal. In some cases, the pitch of the coil may be constant along its length. Alternatively, a first portion of the coil may have a first pitch, and a second portion of the coil may have a second pitch. This variation in pitch may allow for different functional characteristics along the length of the coil, potentially enhancing its effectiveness in different stages of the biological material removal process.

The distal end 610 of the biological material removal device 600 may have various configurations to enhance its effectiveness in interacting with biological material. In some cases, the distal end 610 of the elongated member may be rounded. This rounded configuration may help prevent damage to vessel walls or other delicate tissues during insertion and manipulation of the device.

Alternatively, the distal end 610 of the elongated member may be tapered. A tapered configuration may facilitate easier insertion of the device through narrow passages or through biological material. In some cases, the coil or fiber 608 may have a sharp tip that can hook onto biological material, enhancing the device's ability to capture and remove biological material.

The positioning of the distal end 610 relative to other components of the biological material removal device 600 may vary. In some cases, the distal end 610 of the elongated member may be configured to be offset axially from the proximal end of the shape memory material 604. Alternatively, the distal end 610 of the elongated member may be configured to be coplanar with a proximal end of the shape memory material 604. These different configurations may allow for optimization of the device's performance in various clinical scenarios.

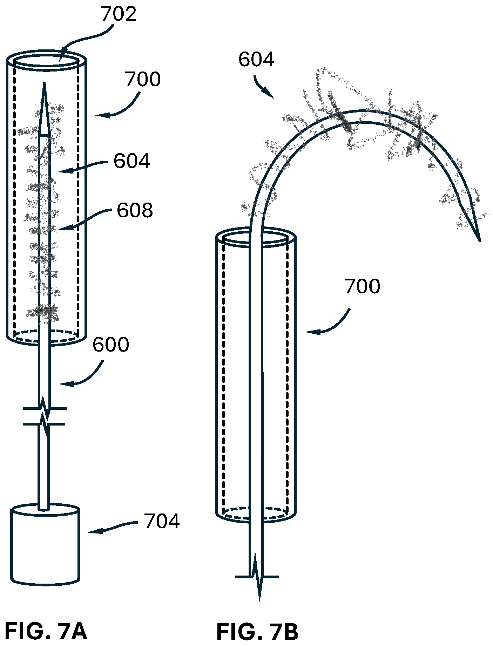

As shown in FIG. 7A and FIG. 7B, the biological material removal device 600 may be designed to work in conjunction with a tubular member 700. The shape memory material 604 with the coil or fiber 608 may be positioned within a lumen 702 of the tubular member 700. This configuration may allow for controlled deployment and retraction of the biological material removal device 600 during procedures. When in the tube, the shape memory material may remain relatively straight (see FIG. 7A), but when outside the tube, the shape memory material may revert to its desired shape (see FIG. 7B).

The combination of the shape memory material 604 and the coil or fiber 608 may provide a versatile and effective means of capturing and removing biological material from within medical devices such as access or introducer sheaths. The various configurations and material options for these components may allow for customization of the biological material removal device 600 to suit different clinical needs and scenarios.

The tubular member 700 may be a key component of the biological material removal system, designed to work in conjunction with the biological material removal device 600. As shown in FIG. 7A and FIG. 7B, the tubular member 700 may have a lumen 702 extending through its length. The lumen 702 may be configured to accommodate the biological material removal device 600, allowing for controlled deployment and retraction during procedures.

In some cases, the tubular member 700 may be composed of one or more polymeric materials. These materials may include, but are not limited to, polyethylene, polyurethane, or polytetrafluoroethylene (PTFE). The choice of material may depend on factors such as flexibility, biocompatibility, and durability required for the specific application.

The dimensions of the tubular member 700 may vary depending on the intended use. In some cases, the outer diameter of the tubular member 700 may range from about 1 mm to 10 mm, while the inner diameter defining the lumen 702 may range from about 0.5 mm to 9 mm. These dimensions may be selected to accommodate the biological material removal device 600 while still allowing for insertion into standard access or introducer sheaths.

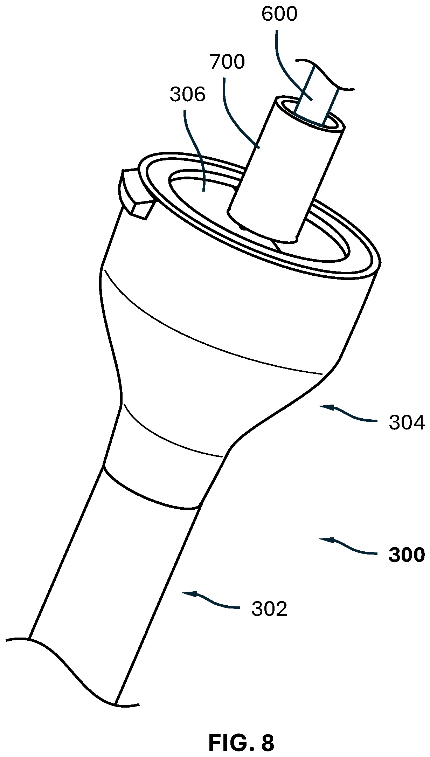

As illustrated in FIG. 8, the tubular member 700 may be configured to extend into an access or introducer sheath 300. In some cases, a distal end of the tubular member 700 may be inserted into a hub 304 of the access or introducer sheath 300, at least partially through a hemostatic valve 306. This configuration may allow the tubular member 700 to provide a pathway for the biological material removal device 600 while maintaining hemostasis.

The tubular member 700 may include a handle or grip 704, as shown in FIG. 7A and FIG. 7B. The handle or grip 704 may be operably coupled to a proximal end of the tubular member 700, facilitating manipulation and control during insertion and withdrawal procedures.

In some cases, the biological material removal device 600 may be deployed through the tubular member 700. The shape memory material 604 with the coil or fiber 608 may be positioned within the lumen 702 of the tubular member 700. When deploying the biological material removal device 600, the coil or fiber 608 may be moved distally through the lumen 702 such that a distal end of the coil or fiber 608 is within or adjacent to biological material.

The movement of the coil or fiber 608 through the lumen 702 may vary. In some cases, when the coil or fiber 608 moves distally, the coil or fiber 608 may move axially without rotation. Alternatively, when the coil or fiber 608 moves distally, the coil or fiber 608 may move axially and rotate at the same time. The specific movement pattern may depend on the design of the biological material removal device 600 and the desired interaction with the biological material.

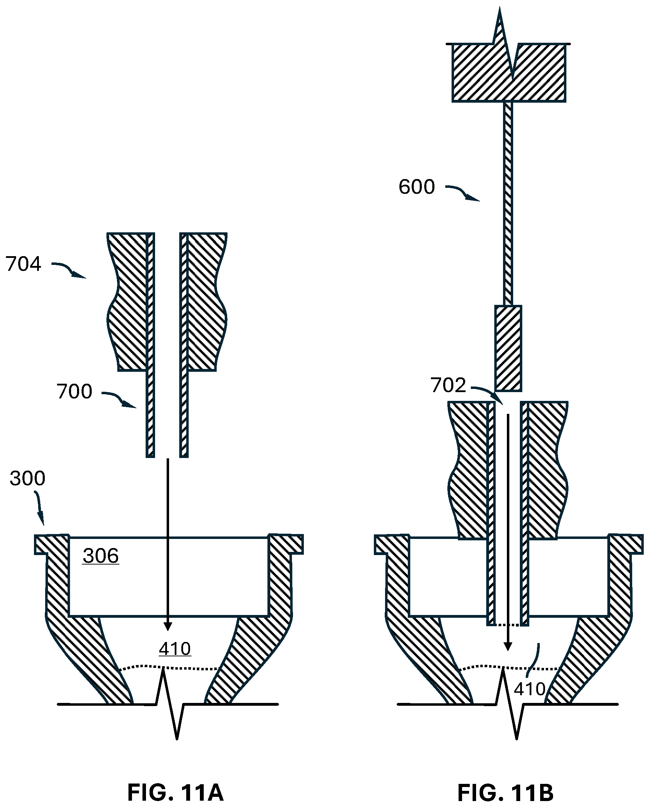

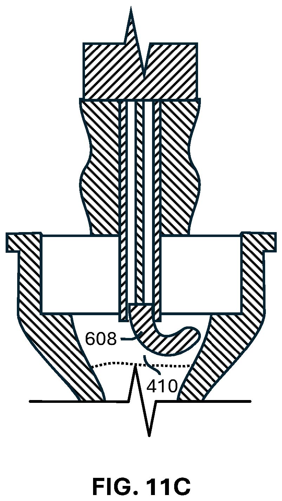

An implementation of this is illustrated in FIGS. 11A-11C. In FIG. 11A, tubular member 700 is inserted into the hub 304, and specifically is inserted through some or all of the hemostatic valve 306 of the hub 304. In FIG. 11B, it is shown that the tubular member may be inserted until the handle or grip 704 prevents the tubular member from being inserted further. As will be appreciated, the tubular member may be inserted any suitable distance. For example, the tubular member may be inserted, and the user may choose to stop insertion before the handle or grip 704 prevents the tubular member from being inserted further. At least a portion of the distal end of the tubular member may extend into the hub, through the hemostatic valve, to allow access to the biological material 410. The coil or fiber 608 may then be inserted into the hub, through the lumen 206 of the tubular member 204. In FIG. 11C, it is shown that when the coil or fiber 608 is within or adjacent to the biological material 410, the coil or fiber 608 can then be rotated (e.g., around a central axis) to contact/capture the biological material.

The tubular member 700 may be designed to allow for the passage of dislodged biological material. In some cases, the lumen 702 of the tubular member 700 may provide a channel through which dislodged biological material can exit the hub 304 with the flow of pressurized blood. This feature may facilitate the removal of biological material from the access or introducer sheath 300 without the need for complete withdrawal of the sheath.

In some cases, the distal end of the coil or fiber 608 may be configured to extend distally beyond a distal end of the tubular member 700 when fully inserted. This configuration may allow the coil or fiber 608 to interact with biological material within the access or introducer sheath 300 while the tubular member 700 remains in place.

The tubular member 700 and the biological material removal device 600 may be provided as part of a kit. In some cases, the kit may include the tubular member 700 configured to extend into an access or introducer sheath 300, and a coil or fiber 608 configured to slidably extend through the lumen 702 of the tubular member 700. This kit configuration may provide flexibility for different biological material removal scenarios or serve as backup equipment.

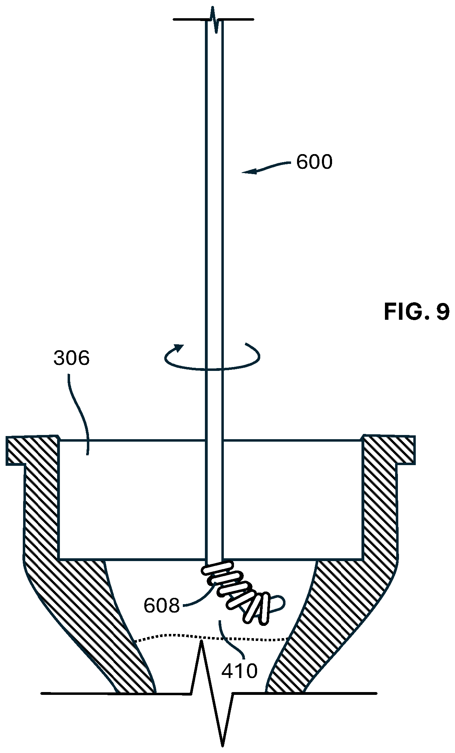

FIG. 9 illustrates a section view of the biological material removal system showing the interaction between key components during a biological material removal procedure. The biological material removal device 600 may be configured to interact with biological material 410 within an access or introducer sheath 300.

In some cases, the biological material removal device 600 may be inserted through the hemostatic valve 306 of the hub 304. The coil or fiber 608 of the biological material removal device 600 may be moved distally through the lumen of the tubular member such that a distal end of the coil or fiber 608 is within or adjacent to the biological material 410.

In some instances, inserting the tube of the biological material removal device may cause the biological material to exit the sheath. As a simple example, biological material caught in leaflets of the hemostatic valve may exit the sheath, driven by the backbleed pressure induced by the insertion of the tube. However, in other instances, the biological material may remain in the sheath or hub after insertion,

The biological material removal device 600 may be designed to capture the biological material 410 by rotating the elongated member. In some cases, the elongated member may be twirled 360 degrees to sweep the inner wall of the hub 304. This rotational movement may allow the coil or fiber 608 to engage with and entangle the biological material 410.

The rotation of the biological material removal device 600 may be achieved through various means. In some cases, the rotation may be manually performed by manipulating the handle or grip attached to the proximal end of the elongated member. Alternatively, the rotation may be electronically controlled using the motor or actuator operably coupled to the proximal end of the elongated member.

After capturing the biological material 410, the biological material removal device 600 and the captured biological material 410 may be withdrawn from the access or introducer sheath 300. In some cases, the withdrawal process may involve pulling the elongated member and the attached biological material 410 through the hemostatic valve 306 of the hub 304.

The withdrawal of the biological material removal device 600 and the biological material 410 may be coordinated with the withdrawal of the tubular member. In some cases, the tubular member may be withdrawn from the hub 304 prior to withdrawing the elongated member. This sequential withdrawal may allow for a controlled removal of the biological material removal components.

Alternatively, the tubular member may be withdrawn from the hub 304 simultaneous with or after withdrawing the elongated member. This approach may provide flexibility in the biological material removal procedure, allowing for adaptation to different clinical scenarios.

In some cases, the coil or fiber 608 and the captured biological material 410 may be withdrawn through the lumen of the tubular member. The lumen may provide a pathway for the safe removal of the biological material 410, minimizing the risk of biological material fragmentation or dislodgement during the withdrawal process.

The withdrawal of the biological material removal device 600 and the biological material 410 may be facilitated by the design of the hemostatic valve 306. The hemostatic valve 306 may be configured to maintain a seal around the elongated member during withdrawal, helping to prevent blood loss or air entry into the access or introducer sheath 300.

In some cases, the sensor disposed in or on the tubular member may provide information during the biological material removal process. The information from the sensor may be used to guide the rotation and withdrawal of the biological material removal device 600, potentially enhancing the effectiveness and safety of the procedure.

The biological material removal system may be designed to allow for multiple passes if necessary. In some cases, the biological material removal device 600 may be reinserted through the hemostatic valve 306 to capture additional biological material 410 that may remain within the access or introducer sheath 300 or the hub 304.

The biological material removal system 200 may include various optional features and control mechanisms to enhance its functionality and performance. FIG. 4 illustrates a cross-sectional view of the biological material removal system 200, showing internal components and their arrangement.

In some cases, the biological material removal system 200 may include a motor or actuator 404 operably coupled to a proximal end of the coil 202. The motor or actuator 404 may be configured to control axial movement and/or rotation of the coil 202. This configuration may allow for precise and controlled manipulation of the coil 202 during biological material removal procedures.

The biological material removal system 200 may include circuitry 406 associated with the motor or actuator 404. The circuitry 406 may be designed to control the operation of the motor or actuator 404, allowing for electronic control of the coil's movement and rotation. In some cases, the circuitry 406 may be located in proximity to the motor or actuator 404 within the biological material removal system 200.

A button 408 may be incorporated into the biological material removal system 200. The button 408 may be configured to initiate the movement and rotation of the coil 202. In some cases, pressing the button 408 may activate the motor or actuator 404, causing the coil 202 to move axially, rotate, or perform a combination of these movements.

The biological material removal system 200 may include a sensor 412 disposed in or on the tubular member 204. The sensor 412 may be designed to gather information about the environment within the access or introducer sheath 300 or the surrounding tissue. In some cases, the axial movement and/or rotation of the coil 202 may be controlled based on information from the sensor 412. This feedback mechanism may allow for adaptive control of the biological material removal process.

In some cases, the tubular member 204 and coil 202 may be operably coupled together. This coupling may allow for coordinated movement and control of both components. Alternatively, the tubular member 204 and coil 202 may be free of mechanical or electrical connections between them, allowing for independent manipulation.

The biological material removal system 200 may include a handle or grip 210 operably coupled to a proximal end of the coil 202. Additionally, a handle or grip 212 may be operably coupled to a proximal end of the tubular member 204. These handles or grips may facilitate manual control and manipulation of the coil 202 and tubular member 204.

While electronic control may be implemented in some cases, the biological material removal system 200 may also allow for manual operation. In some cases, moving the coil 202 and rotating the coil 202 may be manually performed. This manual option may provide flexibility in different clinical scenarios or serve as a backup in case of electronic system failure.

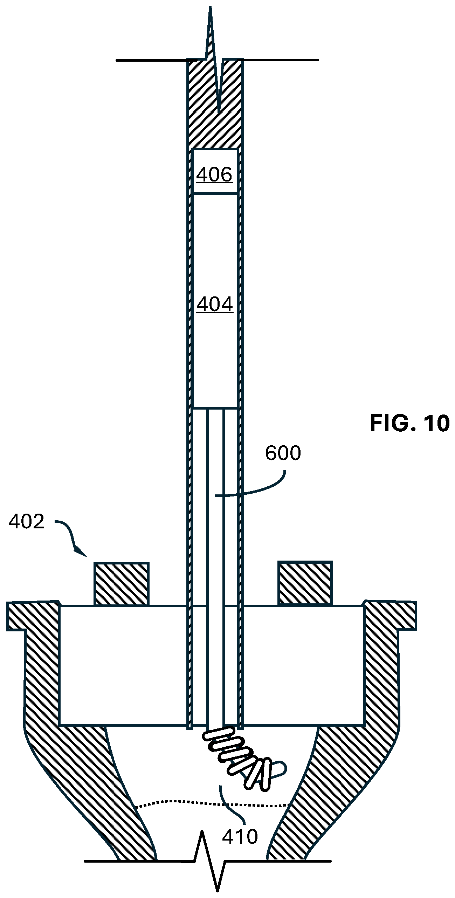

FIG. 10 illustrates a section view of the biological material removal device 600 assembly, which may incorporate similar control mechanisms. In some cases, a motor or actuator 404 may be operably coupled to a proximal end of the elongate member of the biological material removal device 600. The motor or actuator 404 may be configured to control rotation of the elongate member, facilitating the capture and removal of biological material.

The biological material removal device 600 may include a handle or grip operably coupled to a proximal end of the elongated member. This handle or grip may allow for manual rotation of the elongate member in some cases.

A tab or protrusion 402 may extend from the outer structure of the biological material removal system 200 or the biological material removal device 600. The tab or protrusion 402 may serve various functions, such as providing a grip point for manual manipulation or acting as an indicator of device orientation. The tabs or protrusions may also be used to ensure the device is positioned correctly and not over- or under-inserted.

In some cases, rotating the elongate member of the biological material removal device 600 may be electronically controlled. This electronic control may be initiated by pressing a button, similar to the operation of the coil 202 in the biological material removal system 200.

The integration of these optional features and control mechanisms may allow for precise manipulation of the coil 202 or other elongate members within the access or introducer sheath 300. The ability to control axial movement and rotation electronically or manually may enhance the effectiveness of biological material removal procedures while potentially reducing procedural complexity and operator fatigue.

Referring to FIG. 5, the method of biological material removal 500 may include inserting 504 a distal end of the elongate member into a hub 304 of an access or introducer sheath 300 (e.g., through a hemostatic valve 306), where the distal end of the elongate member includes a coil or fiber 608. This may include moving the coil or fiber distally through the hub such that the coil or fiber is within or adjacent to biological material 410.

The method may include capturing 506 the biological material by rotating the elongate member. This may be done manually (e.g., by manually rotating a handle coupled to a proximal end of the elongate member), or electronically (e.g., by having a motor rotate the elongate member).

The method may include withdrawing 508 the elongate member and the biological material from the access or introducer sheath.

The method may include inserting a distal end of a tubular member into the hub prior to inserting the elongate member. The elongate member can be inserted through the tubular member (see, e.g., FIG. 7A), where the shape memory material at a distal end of the device is kept relatively straight when within the tube, but when the elongate member is moved distally, and the shape memory material is no longer within the tubular member, the shape memory material can revert to its desired shape (see, e.g., FIG. 7B). As such, inserting 504 a distal end of an elongate member into the hub may include inserting the distal end of the elongate member through the tubular member.

The method may include withdrawing the tubular member from the hub prior to withdrawing the elongate member. The method may alternatively include withdrawing the tubular member from the hub simultaneous with or after withdrawing the elongate member.

The biological material removal system and/or biological material removal device may be provided as part of a kit configuration. In some cases, the kit may include multiple components designed to work together for effective biological material removal from medical devices such as intravascular access sheaths.

As illustrated in FIG. 8, the kit may comprise a biological material removal device 600 and a first tubular member 700. The first tubular member 700 may have a proximal end and a distal end, with a lumen extending therethrough. In some cases, the first tubular member 700 may be configured to extend into an access or introducer sheath 300. The biological material removal device 600 may be configured to be slidably received through the lumen of the first tubular member 700.

The kit may further include additional components to enhance versatility and functionality. In some cases, the kit may comprise a coil configured to slidably extend through the lumen of the first tubular member 700. The distal end of the coil may be configured to extend distally beyond a distal end of the first tubular member 700 when the coil is fully inserted into the first tubular member 700. This configuration may allow for targeted interaction with biological material within the access or introducer sheath 300.

In some cases, the kit may further comprise a biological material removal system including a second tubular member. The second tubular member may have a proximal end and a distal end, with a lumen extending therethrough. The second tubular member may be configured to extend into an access or introducer sheath 300, similar to the first tubular member 700.

The biological material removal system included in the kit may also comprise a coil configured to extend at least partially through the lumen of the second tubular member. In some cases, a distal end of the coil may be configured to extend distally beyond a distal end of the second tubular member when the coil is fully inserted into the second tubular member. This arrangement may provide an alternative or complementary biological material removal option within the kit.

The components of the kit may be made from various materials suitable for medical use. In some cases, the tubular members may be composed of polymeric materials such as polyethylene, polyurethane, or polytetrafluoroethylene (PTFE). The coils may be made from materials including, but not limited to, stainless steel, nitinol, or polymer-based materials.

The dimensions of the kit components may vary to accommodate different clinical scenarios. In some cases, the length of the tubular members may range from about 10 cm to 150 cm. The outer diameter of the tubular members may range from about 1 mm to 10 mm, while the inner diameter may range from about 0.5 mm to 9 mm.

The kit may also include additional accessories to support the biological material removal procedure. In some cases, the kit may comprise lubricants, guidewires, or introducer needles compatible with the included components. Instructions for use may also be provided as part of the kit, detailing proper assembly and operation of the biological material removal system.

The configuration of the kit may allow for flexibility in addressing various biological material removal scenarios. The inclusion of multiple tubular members and coils may provide options for different access sheath sizes or biological material characteristics. In some cases, the kit components may be designed for single use, while in other cases, certain components may be reusable after proper sterilization.

The packaging of the kit may be designed to maintain sterility of the components and facilitate easy access during procedures. In some cases, the kit may be packaged in a tray with separate compartments for each component, allowing for organized presentation in the sterile field.

A number of implementations have been described. Nevertheless, it will be understood that various modifications may be made without departing from the spirit and scope of the disclosure. Accordingly, other implementations are within the scope of the following claims.

### Exemplary Embodiments

A1 A method of biological material removal, comprising: inserting a distal end of a tubular member of a biological material removal system into a hub of an access or introducer sheath, at least partially though a hemostatic valve, the tubular member having a lumen extending from a proximal end to a distal end; moving a coil distally through the lumen such that a distal end of the coil is within or adjacent to biological material; capturing the biological material by rotating the coil; and withdrawing the coil and the biological material from the access or introducer sheath.

A2 The method of paragraph A1, further comprising withdrawing the tubular member from the access or introducer sheath.

A3 The method of paragraph A1 or A2, further comprising providing or selecting a biological material removal system.

A4 The method of any one of paragraphs A1 to A3, wherein moving the coil and rotating the coil is manually performed.

A5 The method of any one of paragraphs A1 to A4, wherein moving the coil and rotating the coil is electronically controlled.

A6 The method of any one of paragraphs A1 to A5, wherein moving the coil and rotating the coil is initiated by pressing a button.

A7 The method of any one of paragraphs A1 to A6, wherein when the coil moves distally, the coil moves axially without rotation.

A8 The method of any one of paragraphs A1 to A6, wherein when the coil moves distally, the coil moves axially and rotates simultaneously.

A9 A biological material removal system, comprising: a tubular member having a proximal end and a distal end, and a lumen extending therethrough, the tubular member configured to extend into an access or introducer sheath; and a coil extending at least partially through the lumen, a distal end of the coil configured to extend distally beyond a distal end of the tubular member when the coil is fully inserted into the tubular member.

A10 The biological material removal system of paragraph A9, further comprising a handle or grip operably coupled to a proximal end of the coil.

A11 The biological material removal system of paragraph A9 or A10, further comprising a handle or grip operably coupled to a proximal end of the tubular member.

A12 The biological material removal system of any one of paragraphs A9 to A11, wherein the tubular member and coil are free of mechanical or electrical connections between them.

A13 The biological material removal system of any one of paragraphs A9 to A12, wherein the tubular member and coil are operably coupled together.

A14 The biological material removal system of any one of paragraphs A9 to A13, further comprising a motor or actuator operably coupled to a proximal end of the coil, and configured to control axial movement and/or rotation of the coil.

A15 The biological material removal system of any one of paragraphs A9 to A14, further comprising a sensor disposed in or on the tubular member.

A16 The biological material removal system of paragraph A15, wherein axial movement and/or rotation of the coil is controlled based on information from the sensor.

A17 The biological material removal system of any one of paragraphs A9 to A16, wherein the tubular member is composed of one or more polymeric materials.

A18 The biological material removal system of any one of paragraphs A9 to A17, wherein the coil is composed of one or more polymer materials.

A19 The biological material removal system of any one of paragraphs A9 to A18, wherein a pitch of the coil is constant.

A20 The biological material removal system of any one of paragraphs A9 to A18, wherein a first portion of the coil has a first pitch, and a second portion of the coil has a second pitch.

A21 The biological material removal system of any one of paragraphs A9 to A20, wherein a distal end of the coil has a rounded tip.

A22 The biological material removal system of any one of paragraphs A9 to A20, wherein a distal end of the coil has a tapered tip.

A23 A kit, comprising: a tubular member having a proximal end and a distal end, and a lumen extending therethrough, the tubular member configured to extend into an access or introducer sheath; and a coil configured to slidably extend through the lumen, a distal end of the coil configured to extend distally beyond a distal end of the tubular member when the coil is fully inserted into the tubular member.

A24 A method of biological material removal, comprising: inserting a distal end of an elongate member into a hub of an access or introducer sheath through a hemostatic valve such that a coil or fiber at a distal end of the elongate member is within or adjacent to biological material, the coil or fiber configured to interact with the biological material; capturing the biological material by rotating the elongate member; and withdrawing the elongate member and the biological material from the access or introducer sheath.

A25 The method of paragraph A24, further comprising inserting a distal end of a tubular member into the hub prior to inserting the elongate member, wherein inserting a distal end of an elongate member into the hub includes inserting the distal end of the elongate member through the tubular member.

A26 The method of paragraph A25, further comprising withdrawing the tubular member from the hub prior to withdrawing the elongate member.

A27 **The** method of paragraph A25, further comprising withdrawing the tubular member from the hub simultaneous with or after withdrawing the elongate member.

A28 **The** method of any one of paragraphs A24 to A27, further comprising providing or selecting a biological material removal system.

A29 **The** method of any one of paragraphs A24 to A28, wherein rotating the elongate member is manually performed.

A30 **The** method of any one of paragraphs A24 to A28, wherein rotating the elongate member is electronically controlled.

A31 **The** method of any one of paragraphs A24 to A30, wherein rotating the elongate member is initiated by pressing a button.

A32 A biological material removal device, comprising: an elongated member having at least a distal portion comprised of a shape memory material; and a coil or fiber disposed around an exterior of at least some of the distal portion, the coil or fiber configured to interact with biological material; wherein the biological material removal device is configured to be slidably inserted through a hemostatic valve of an access or introducer hub.

A33 The biological material removal device of paragraph A32, wherein the shape memory material is nitinol.

A34 The biological material removal device of paragraph A32 or A33, wherein the coil or fiber is a polymeric material.

A35 The biological material removal device of paragraph A32 or A33, wherein the coil or fiber is a metal.

A36 The biological material removal device of any one of paragraphs A32 to A35, further comprising a handle or grip operably coupled to a proximal end of the elongated member.

A37 The biological material removal device of any one of paragraphs A32 to A36, wherein a distal end of the elongated member is rounded.

A38 The biological material removal device of any one of paragraphs A32 to A36, wherein a distal end of the elongated member is tapered.

A39 The biological material removal device of any one of paragraphs A32 to A38, wherein a distal end of the elongated member is configured to be offset axially from a proximal end of the shape memory material.

A40 The biological material removal device of any one of paragraphs A32 to A38, wherein a distal end of the elongated member is configured to be coplanar with a proximal end of the shape memory material.

A41 The biological material removal device of any one of paragraphs A32 to A40, wherein the elongated member and coil are free of mechanical or electrical connections between them.

A42 The biological material removal device of any one of paragraphs A32 to A41, wherein the elongated member and coil are operably coupled together.

A43 The biological material removal device of any one of paragraphs A32 to A42, further comprising a motor or actuator operably coupled to a proximal end of the elongated member, and configured to control rotation of the elongated member.

A44 A kit, comprising: a biological material removal device of any one of paragraphs A32 to A43; and first tubular member having a proximal end and a distal end, and a lumen extending therethrough, the first tubular member configured to extend into an access or introducer sheath, the biological material removal device configured to be slidably received through the lumen.

A45 The kit of paragraph A44, further comprising a coil configured to slidably extend through the lumen, a distal end of the coil configured to extend distally beyond a distal end of the first tubular member when the coil is fully inserted into the first tubular member.

A46 The kit of paragraph A44 or A45, further comprising a biological material removal system including: a second tubular member having a proximal end and a distal end, and a lumen extending therethrough, the second tubular member configured to extend into an access or introducer sheath; and a coil configured to extend at least partially through the lumen, a distal end of the coil configured to extend distally beyond a distal end of the second tubular member when the coil is fully inserted into the second tubular member.

## Claims

1. A biological material removal system, comprising:
a tubular member having a proximal end and a distal end, and a lumen extending therethrough, the tubular member configured to extend into an access or introducer sheath; and
a coil extending at least partially through the lumen, a distal end of the coil configured to extend distally beyond a distal end of the tubular member when the coil is fully inserted into the tubular member.

2. The biological material removal system of claim 1, further comprising a handle or grip operably coupled to a proximal end of the coil.

3. The biological material removal system of claim 1 or 2, further comprising a handle or grip operably coupled to a proximal end of the tubular member.

4. The biological material removal system of any of claims 1 to 3, wherein the tubular member and coil are free of mechanical or electrical connections between them.

5. The biological material removal system of any of claims 1 to 4, wherein the tubular member and coil are operably coupled together.

6. The biological material removal system of any of claims 1 to 5, further comprising a motor or actuator operably coupled to a proximal end of the coil, and configured to control axial movement and/or rotation of the coil.

7. The biological material removal system of any of claims 1 to 6, further comprising a sensor disposed in or on the tubular member, wherein axial movement and/or rotation of the coil is preferably controlled based on information from the sensor.

8. The biological material removal system of any of claims 1 to 7,
wherein the tubular member and/or the coil is composed of one or more polymer materials.

9. The biological material removal system of any of claims 1 to 8, wherein a pitch of the coil is constant, or wherein a first portion of the coil has a first pitch, and a second portion of the coil has a second pitch; and/or
wherein a distal end of the coil has a rounded tip or a tapered tip.

10. A kit, comprising:
a tubular member having a proximal end and a distal end, and a lumen extending therethrough, the tubular member configured to extend into an access or introducer sheath; and
a coil configured to slidably extend through the lumen, a distal end of the coil configured to extend distally beyond a distal end of the tubular member when the coil is fully inserted into the tubular member.

11. A biological material removal device, comprising:
an elongated member having at least a distal portion comprised of a shape memory material; and
a coil or fiber disposed around an exterior of at least some of the distal portion, the coil or fiber configured to interact with biological material;
wherein the biological material removal device is configured to be slidably inserted through a hemostatic valve of an access or introducer hub.

12. The biological material removal device of claim 11, wherein the shape memory material is nitinol, and/ or wherein the coil or fiber is a polymeric material or metal.

13. The biological material removal device of claim 11 or 12, further comprising a handle or grip operably coupled to a proximal end of the elongated member, and/ or wherein a distal end of the elongated member is rounded or tapered.

14. The biological material removal device of and of claims 11 to 13, wherein a distal end of the elongated member is configured to be offset axially from a proximal end of the shape memory material or to be coplanar with a proximal end of the shape memory material.

15. The biological material removal device of any of claims 11 to 14, wherein the elongated member and coil are free of mechanical or electrical connections between them; and/ or wherein the elongated member and coil are operably coupled together; and/ or further comprising a motor or actuator operably coupled to a proximal end of the elongated member, and configured to control rotation of the elongated member.
